Europäisches Patentamt

European Patent Office

Office européen des brevets

(11) Publication number: **0 430 395 A1**

# EUROPEAN PATENT APPLICATION

(21) Application number: 90307137.1

(51) Int. Cl.5: **C12N 11/08**, C12N 11/04

(22) Date of filing: 29.06.90

(30) Priority: 24.11.89 US 440697

(43) Date of publication of application:
05.06.91 Bulletin 91/23

(84) Designated Contracting States:
AT BE CH DE DK ES FR GB GR IT LI LU NL SE
Bulletin

(71) Applicant: KINGSTON DIAGNOSTICS, L.P.
2235-B Route 130
Dayton, New Jersey 08810(US)

(72) Inventor: Thakore, Yatin B.
11 Rues Lane
East Brunswick, New Jersey 08816(US)

(74) Representative: Ablewhite, Alan James et al
MARKS & CLERK 57/60 Lincoln's Inn Fields
London WC2A 3LS(GB)

(54) Stabilized dispersed enzymes.

(57) Enzymes are immobilized, stabilized and evenly dispersed without destroying their activity by sequestering them within micropores of a solid carrier structure and filling the pores with a water-immiscible organic liquid, in which the enzyme remains active. The preferred solid carrier is a microporous membrane. The system is manufactured by providing a solid carrier having a microporous surface whose pores contain an aqueous solution, allowing an aqueous solution of enzyme to equilibrate with the solution in the pores, causing a controlled partial collpase of the pores to effectively trap the enzymes, and replacing the liquid in the pores with a water-immiscible organic liquid. The system can be used to assay for or produce an organic compound. It also can be used for slow release of an enzyme.

EP 0 430 395 A1

## STABILIZED DISPERSED ENZYMES

## BACKGROUND OF THE INVENTION
### 1. Field of the Invention

This invention relates to enzymatic processes, e.g., for assaying or synthesizing specific organic compounds.

Enzymatic reactions are widely used in various biomedical and industrial applications such as assaying or synthesizing specific organic compounds. To facilitate enzyme handling and recovery, enzymes have been immobilized on insoluble supports, including membranes. Techniques for immobilization include chemical cross-linking, covalent binding to supports, physical entrapment or adsorption, or a combination of physical and chemical processes.

To be functional, enzymes must retain their natu rally occuring folding pattern which establishes the configuration or structure necessary for enzymatic activity. Denaturization of the enzyme (alteration of its folding pattern accompanied by loss of activity) can result from prolonged storage, heat, and other environmental factors. Many immobilization methods, particularly covalent binding and cross-linking, have been proposed to preserve the native conformation of the enzymes, but such methods themselves may contribute to enzyme inactivation.

Kazandjian et al., Biotech. and Bioeng. XXVIII:417-421 (1986) disclose a method of precipitating two enzymes, horseradish peroxidase and cholesterol oxidase, onto a glass powder. First they form an aqueous slurry of enzyme and powder, and-then they dry the slurry to obtain "visibly dry (free-flowing) beads." They add the resulting beads to enzyme substrate (p-amisidine) dissolved in various solvents, and conclude that the reaction proceeds fastest in

"very hydrophobic, water immiscible solvents that evidently do not strip the essential water from the enzyme even if no exogenous water is added (on top of that brought in with $H_2O_2$) . . . . (Even] in toluene and other highly hydrophobic organic solvents, a certain amount of water present is required."

They attribute loss of enzymatic activity in less hydrophobic, more water-miscible solvents to stripping of critical water molecules from the enzyme.

Klibanov, Science 219: 722-72-7 (1983) discloses various strategies for enzyme stabilization, including attaching the enzyme to a support by multiple links to avoid unfolding, and encapsulating the enzyme in membranes that are impermeable for enzymes, but permeable for low molecular weight substrates and products. Entrapment in microcapsules is accomplished by "interfacial polymerization, liquid drying or phase separation." Entrapment in liposomes or in hollow fibers is also disclosed.

Zaks and Klibanov, Science, 224:1249-1251 (1984) and Zaks and Klibanov, Proc. Natl Acad. Sci. 82:3192-3196 (1985) disclose that porcine pancreatic lipase, yeast lipase, and mold lipase retain their activity in nearly anhydrous organic solvents. They further disclose that, while water is essential for maintenance of activity of these enzymes, it also participates in inactivation processes, particularly thermal inactivation. They further disclose that enzymes are more heat-stable in organic, water-immiscible solvents.

## SUMMARY OF THE INVENTION

I have discovered that enzymes can be stabilized and evenly dispersed without destroying their activity by sequestering the enzyme at least at the surface of a solid carrier structure having micropores and filling the pores with a water-immiscible organic liquid, in which the enzyme remains active. The preferred solid carrier is a microporous membrane.

## BRIEF DESCRIPTION OF THE DRAWINGS

The present invention is more fully understood when the specification herein is taken in conjunction with the appended drawings, wherein:

Figure 1 is a flow diagram of the steps for manufacturing an enzyme system.
Figure 2 is a graph demonstrating the system of example 1.
Figure 3 is a graph demonstrating the system of example 2.
Figure 4 is a graph demonstrating the system of

## DETAILED DESCRIPTION OF THE PRESENT INVENTION

This invention teaches a novel, simple and practical way of achieving a uniform dispersion of one or more enzymes in a pre-formed polymer matrix such as a membrane and stabilization of such uniformly dispersed enzymes by essentially non-polar solvents as described by Kazandjian et al (supra) or Zaks et al (supra).

The method of achieving such a uniform dispersion falls in a class of methods of enzyme immobilization described as enzyme entrapment but differs from conventional entrapment methods in that it involves no cross-linking or polymerization reaction and thereby offers more flexibility in the choice of membrane materials and the enzymes themselves. Additionally, this method offers a way to stabilize the enzymes subsequent to entrapment.

The current state of art of immobilization of enzymes is summarized by Klibanov, (supra). As described therein, they can be divided into five classes:

a.) Covalent attachment; b.) Adsorption of enzymes on solid supports, typically ion exchange supports; c.) Entrapment of enzymes in polymeric gels; d.) Cross-linking of enzymes with trifunctional reagents; and, e.) Encapsulaton of enzymes.

The intent behind immobilization is usually to stabilize the enzymes against denaturation.

A common mechanism of denaturation is when the enzymes (which are essentially high molecular weight proteins with catalytic activity) lose their catalytic capability by losing their three dimensional strucure via chain unfolding which is responsible for its catalytic action The intent of immobilization is to restrict the movement of enzyme chains by securing at least a portion of enzyme chains to a support and/or by confining them in cage-like structures on a molecular scale such that they are much more restricted in their movements and the denaturation is slowed down or in other words enzymes are stabilized.

In many methods however such goals are not always realized. Particularly the methods that rely on covalent attachment, conventional entrapment and cross-linking with bifunctional reagents employ chemical reactions and often many enzymes don't survive the treatment. A large fraction of enzyme activity could be lost during the treatment itself. For each enzymes, for each type of reaction, and for each support usually extensive optimization need to be done.

The above methods and the physical adsorption methods additionally are generally unsuitable for applications which invovle multiple enzymes. Many diagnostics applications e.g. cholesterol, triglycerides, etc. use a one cascade of enzymes where product of one enzymatic reactions are a substrate for the next. When the enzymes are bound as in the above methods they usually will not couple with each other easily and the ration of various enzymes necessary for proper performance can also not be adjusted easily without extensive optimization experiments.

Encapsulation methods although useful for multiple enzymes also have some drawbacks that during encapsulation, the enzymes do come in contact with the organic solvents which often harmful to the enzymes.

In preferred embodiments, the organic liquid is characterized by a dipole moment less than 2.5 Debye units, a dielectric constant less than 20, and a boiling point at atmospheric pressure of at least about 40°C but preferably over about 75°C. The system is particularly useful for enzymes such as cholesterol oxidase, whose substrates are insoluble or sparingly soluble (less than about 10g/1) in water under physiological conditions. Preferably the liquid content of the carrier is at least 90 percent water-immiscible organic liquid.

A second aspect of the invention generally features a method of making a stabilized, evenly dispersed enzyme system, by providing a solid carrier having a microporous structure whose pores contain an aqueous solution, allowing an aqueous solution of enzyme to equilibrate with the solution in the pores, and replacing the liquid in the pores with a water-immiscible organic liquid. Preferable, after the equilibration of the enzyme pores are subjected to controlled partial collapse. By partial collapse, I mean that the pores are not eliminated, but they are collapsed sufficiently to effectively trap the enzyme. The preferred method of effecting controlled partial collapse and replacement of the aqueous liquid is to dry the carrier surface to remove water, introduce a water miscible organic liquid into the pores, and then contact the carrier with the immiscible organic liquid, allowing replacement by diffusion. Preferably, the largest membrane pores initially have a diameter in the range of 10 Angstroms to 100 microns. After controlled collapse, the pore size is reduced to sequester the enzyme (or cell debris containing the enzyme) to on the order of between 5 Angstroms and 10 microns.

The invention offers improved enzyme stability, e.g., as manifested by shelf-life in a diagnostic kit; moreover it substantially improves heat tolerance of the enzyme, e.g. so that its catalytic reaction can be performed at temperatures well above ordinary physiological temperatures. Also, the invention provides an seven dispersion of enzyme without formation of clumps or precipitation, which limit surface area contact with the reaction medium. The system is produced under mild conditions so as to preserve enzymatic activity. The system can also be used for enzymes having water soluble substrates, by contacting the enzyme-containing membrane with substrate dissolved in an aqueous phase. Moreover, the system can be used to react substances in aqueous media over a wide pH range, because the organic liquid insulates the membrane from the pH of the surrounding aqueous medium.

The invention also can be used to provide sustained controlled release of active enzyme over time, as described more fully below.

The Enzyme System

Enzymes used in the above-described system may be virtually any known enzyme, but preferred enzymes are those catalyzing reaction of a lipophilic substance, i.e., one that prefers non-polar, hydrophobic water-immiscible liquids over polar liquids such as water; i.e. the substance should be sparingly (if at all) soluble in water, but easily dissolved in the water-immiscible organic liquid chosen.

The organic liquid or solvent used could be any apolar organic solvent which is immiscible with water. In general, the solubility of water in these organic solvents solvents should be less than 10% and preferably less than 1% by weight. The dipole moment of these solvents in general will be between 0 and 2.5 and preferable under 1 Debye unit. The dielectric constants of these solvents in general will be under 20 and preferably under 10. It will be preferable to have solvents with relatively high boiling points specifically for applications invoving higher temperatures. Examples of suitable solvents in this class include toluene, benzene, xylene, hydrocarbons, oils, higher alcohols etc. The organic liquid can be readily selected for a given enzyme as described herein. The final organic liquid content of the membrane should be over 90% of the total liquid in the membranes and preferable over 98% of the total liquid.

The preferred carriers are membranes, although other forms of carrier could be used. Specifically, the membrane can be in any convenient form such as flat sheet, hollow fibers, tubes, sponges or even porous rods or fabricated forms from basic membrane structures. Membranes with larger pores in the range of 10 Angstroms - 100 micron and/or initial void volumes between 20-97% of wet volume are suitable for these procedures, with pore sizes in 10 Angstroms - 5 micron and/or initial void volumes between 50-90% being the most preferred range.

The two primary requirements of membranes for such a procedure are that: i) the initial pore sizes of the membranes are large enough to allow the enzymes of interest to enter the membrane matrix by diffusion (in the case of asymmetric membranes, at least the pores on the more open spongy side are large enough for the 15 enzymes to diffuse into the membrane matrix); and ii) the water (or other non-solvent) in the pores is in its non-equilibrium state, such that the pores can be collapsed irreversibly upon drying. These requirements can be easily met by membranes synthesized from a wide variety of relatively hydrophobic polymers or their derivitives, as described below. Also the membrane should be resistant to the organic solvent of interest.

As noted, the initial water (non-solvent) content should be such that, upon drying or evaporation of the non-solvent, the pores will collapse irreversibly. This requirement is easily met by membranes fabricated from a wide variety of relatively hydrophobic polymers, copolymers or their derivatives such as nylons, polyesters, polysulfones, polyacrylonitriles, polycarbonates, polyvinylchlorides, cellulose esters etc. When solutions of these membranes are cast, spun or extruded and allowed to coagulate in a non-solvent bath or in atmospheric humidity, the membranes contain a high fraction of non-solvent, but upon drying the pore-structure generally collapse irreversibly such that they will not regain the original amount of water or non-solvent upon rewetting.

Alternatively, the membrane could be hydrophilic such that the pores would collapse and the membranes would deswell when water or hydrophilic solvent is replaced by non-polar solvents. Such requirements are also easily met by a number of common polymers such as poly-HEMA or their derivatives, cellulosic or their derivatives, hydrolyzed polyacrylonitrile or their derivatives, collagen, polyvinyl alcohol of their derivatives, etc.

The choice of hydrophobic or hydrophilic polymer matrices depends upon the application. In the case of hydrophilic membranes, the entrapment as described above will be due to the desolvation and consequent partial collapse of the membrane structure in presence of the organic liquids. When such membranes are used in contact with aqueous liquids, e.g., as in diagnostic strips, the membranes would gradually reswell and water will preferentially displace the entrapped organic liquids and cause some of the immobilized enzymes to leach into the analate solution. Such loss would be of little consequence in the case of disposal diagnostic strips but is not desirable for applications requiring continuous processes as for example in bioreactors.

For continuous processes conducted in contact with aqueous or hydrophilic media, hydrophobic membrane matrices which undergo permanent shrinkage of pores will be more desirable. The hydrophilic membrane matrices, however are preferred for controlled release of enzymes, because the enzymes would be stable for a long time but could be released slowly into external aqueous media when contacted by such media. Such systems are useful for example in enzymatic processes involving in situ clean up of soil or water contamination.

Enzyme stabilization in hydrophilic matrices can also be used as a storage device to keep the enzymes active until needed. This would be particularly useful for in-field applications where it may not be practicable to have refrigeration. At the time of use, stabilized enzymes trapped in hydrophilic matrices can release the enzymes into outside aqueous solution. Such stabilized enzymes therefore can also be used with applications requiring hydrophilic substrates and/or cofactors, both of which are largely insoluble in the organic liquids.

Manufacture

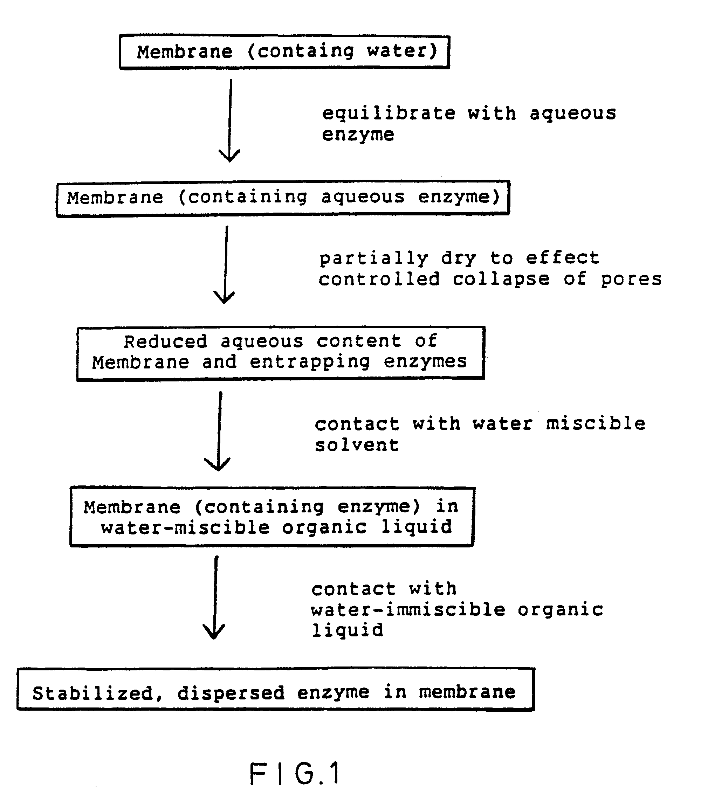

One convenient way to manufacture these stabilized membranes under mild conditions (shown in Fig. 1) is to start with a suitable synthetic membrane containing water or aqueous buffer solution in its pores and with pore-sizes large enough to accomodate the enzyme molecules. The membrane is next placed in a buffered solution of enzyme at its optimum pH and the enzyme is allowed to diffuse into the membrane matrix. After the diffusional exchange, the membrane is partially dried either by gently squeezing the membrane between paper towels or by subjecting it to vacuum. The membrane is of such a type that this partial drying causes partial collapse of the pores, thereby effectively entrapping the enzyme molecules within the pores.

After the entrapment step, the membrane is next placed in a water miscible organic solvent such as acetone, ethanol, etc., and the water in the membrane is exchanged via diffusional exchange. Once this exchange is complete, the enzyme-entrapped membrane is further placed into the desired organic liquid. The diffusional exchange causes the enzyme molecules to be surrounded by the appropriate organic liquids. Since the enzymes are effectively trapped within the pores or void spaces of the polymer matrix, they do remain well-dispersed during the diffusional exchange. The hydrophilicity of parts of the enzyme will result in a small amount of bound water enveloped around the enzyme molecules. Based on current theoretical understanding, it appears that the bound water around enzyme molecules ensures their normal functioning, and the hydrophobic medium surrounding them establishes hydrophobic interaction to prevent unfolding of the enzyme molecules.

Although the above method is a gentle and straight-forward method for making such membranes, there are a variety of other common methods of immobilization which are familiar to those in the field, including cross-linking and entrapment, covalent binding and entrapment, etc. These methods generally could be used for the present invention, but the particular method chosen should be selected to avoid damage to the enzymes. The membrane pores or void spaces should be filled with appropriate organic solvent as soon as possible, subsequent to immobilization.

Finally, even though the present invention describes stabilization of enzymes which are isolated and not a part of living or dead cells, it is possible to prepare such membranes from cell fragments or even whole cells without isolating the enzymes from the cells, The use of the invention is not limited by whether the enzymes are in isolated and purified form or part of cell fragments and therefore in crude, unpurified forms.

The following examples illustrate the invention and they are not to be construed as limitations on it.

Example 1:

Microporous membranes were made by dissolving polyacrylonitrile (molecular weight 150,000) in DMSO at a concentration of 6% by weight, and the solution was filtered through 5 micron stainless steel wire-mesh. The filtered solution was cast on a glass plate with a casting knife resulting in a 10 mil (250) thick solution layer and allowed to coagulate at 700°F and between 70-75% humidity. The polymer solution coagulated in 2-3 hours, giving a microporous membrane with maximum pore-size of 0.8 microns as judged by bubble point. The water content of this membrane was 94% by weight.

The membrane was washed with water for several days and punched into discs of 25 mm diam eter. These discs were then equilibrated overnight with 0.1 M solution of phosphate buffer at pH 7 at 4°C in a refrigerator, and the next day they were equilibrated in a enzyme solution containing cholesterol esterase, cholesterol oxidase (microbial) and horseradish peroxidase at pH 7 in 0.1 phosphate buffer at 4°C. The concentrations of the three enzymes in the buffer solution were 237.5 units/liter, and 71,250 units/liter respectively.

After allowing about 5 hours for tne diffusional exchange, the enzyme-entrapped membrane discs were partially dried by placing them between Kimwipes® (Kimberly-Clark Corp.) in stacks of two, and lightly squeezing them for 5 minutes. During this time, the water content dropped from 94% to between 50 and 70%, and the pores shrank sufficiently to retain even molecules under 10,000 daltons molecular weight.

After this partial collapse of the pores, the films were placed in acetone for about 2 hours, during which time acetone was replaced with fresh acetone at least four times. After this acetone exchange, the enzyme-containing discs were divided into 3 different lots: One lot was placed in dodecane, one lot in 1-heptanol, and one lot in toluene. A few films after partial drying were saved as controls without subjecting them to acetone or solvent exchange. The solvents also contained chromophores and activator in the following concentration: 4-aminoantipyrine at 4.9 m mol/liter; phenol 106.3 m mol/liter and sodium salt of taurocholic acid of 150 mg/liter. (These reagents were not fully soluble in dodecane.) The films were allowed to stay in the solvents for 2 days at $4^\circ$ C during which time fresh solvent was exchanged.

After two days the enzyme containing films were removed from solvent, surface dried, and stored in the refrigerator at $4^\circ$ C until further needed. These films were tested for their ability to detect total cholesterol.

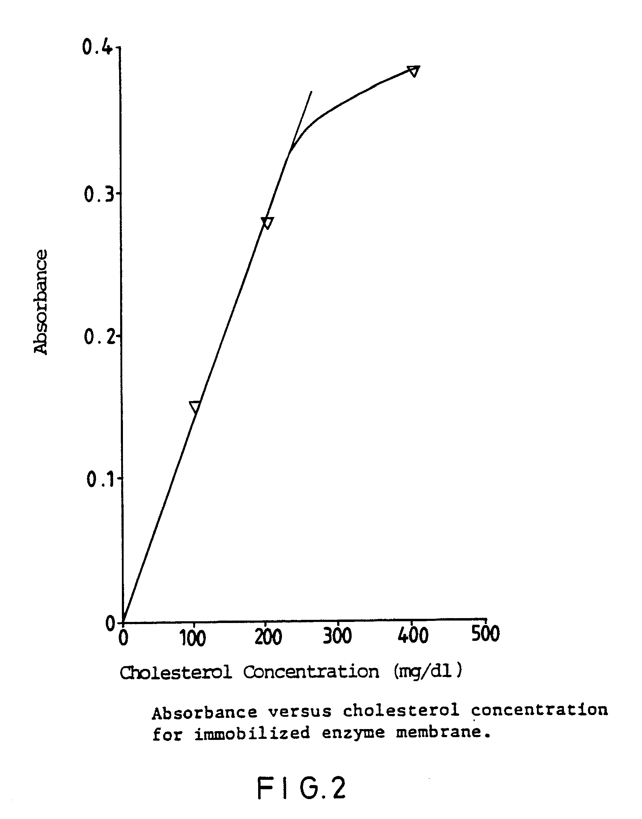

The response to cholesterol concentration was obtained in the following manner: To an enzyme film in a vial, 1 ml of 6% TChA (taurochloric acid sodium salt) solution in 0.1 M phosphate buffer and .04 ml of cholesterol standard solution (between 100 - 400 mg/dl cholestrol ) were added and the vial was incubated at $37^\circ$ C for an hour. The resultant solution in the vial was diluted fourfold with DI water and resultant pinkish color observed against a blank at 500 nm in a visible spectrophotometer. The blank had identical composition except, instead of cholesterol, de-ionized water added. The response was proportional to cholestrol concentration, with toluene films showing the strongest color. The response of toluene film is shown in Figure 2.

Example 2:

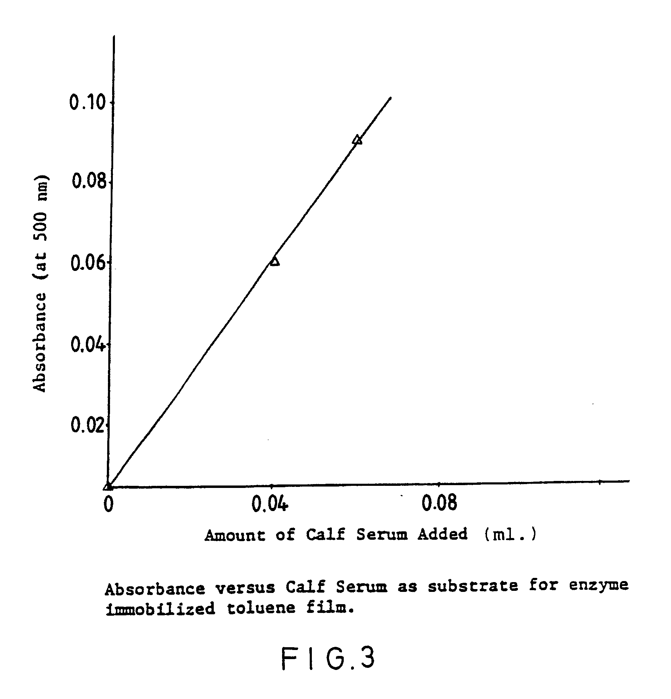

Two toluene films of Example 1 were taken in separate vials and to these 1 ml of 6 g% TChA solution in 0.1 molar phosphate buffer was added followed by 0.04 and 0.08 ml of calf serum respectively. The vials were then incubated for 1 hour at $37^\circ$ C and the solutions were diluted fourfold with DI water. The resultant pinkish colors were measured at 500 nm against a blank (treated identically but without the serum. The response is shown in Figure 3 is proportional to the amount of calf serum. The cholesterol in the calf serum was determined to be 55 mg/dl as judged from the results of Figure 2.

Example 3:

Ultrafiltration membranes were made by dissolving polyacrylonitrile in DMSO at a concentration of 6% by weight and the solution filtered through 5 micron stainless steel wire mesh. The filtered solutions were cast on glass plates with a doctor blade, with a casting thickness of 10 mils and immediately coagulated in DI water at room temperature. The resultant membranes were of ultrafiltration type, with a shiny skin and porous substructure. The molecular weight cut-off was about 100,000 but the pores on the underside were large enough to allow blue dextran (mol. wt. 2,000,000 daltons) to penetrate through.

The procedure for enzyme immobilization in in this membranes was identical as described in Example 1 and the results were similar.

Example 4:

The films of examples 1 and 3 were heated in vials at $60^\circ$ C for 3 hours. The films containing immobilized enzyme in the aqueous buffer as well as free buffered enzyme solutions were also heated as controls. The free enzyme solutions were heated for only 1 hour at $60^\circ$ C.

After heating, the vials were allowed to cool to room temperature and 0.25 ml chromophore and 0.75 ml of 6% TChA activator solution in phosphate buffer were added to the films. In the case of free enzyme solutions, to 0.5 ml of enzyme solution, 0.25 ml each of the chromophore and activator solutions were added. To each of these solutions, 0.04 ml of 400 mg/dl cholesterol standard was added and the solution was incubated at $37^\circ$ C for 45 minutes. As a blank, 0.25 ml chromophore and 0.75 ml activator solution was heated in an identical manner followed by five fold dilution with DI water. The chromophore solution had 4.9 m mol/liter 4 aminoantipyrine and 106.3 m mol/liter of phenol in 0.1 phosphate buffer.

The results are shown in Table 1 which clearly shows that the aqueous free enzymes as well as aqueous immobilized enzymes are virtually inactive whereas the organic solvent immobilized films retain their activities. Toluene films have the highest thermal stabilities.

Example 5:

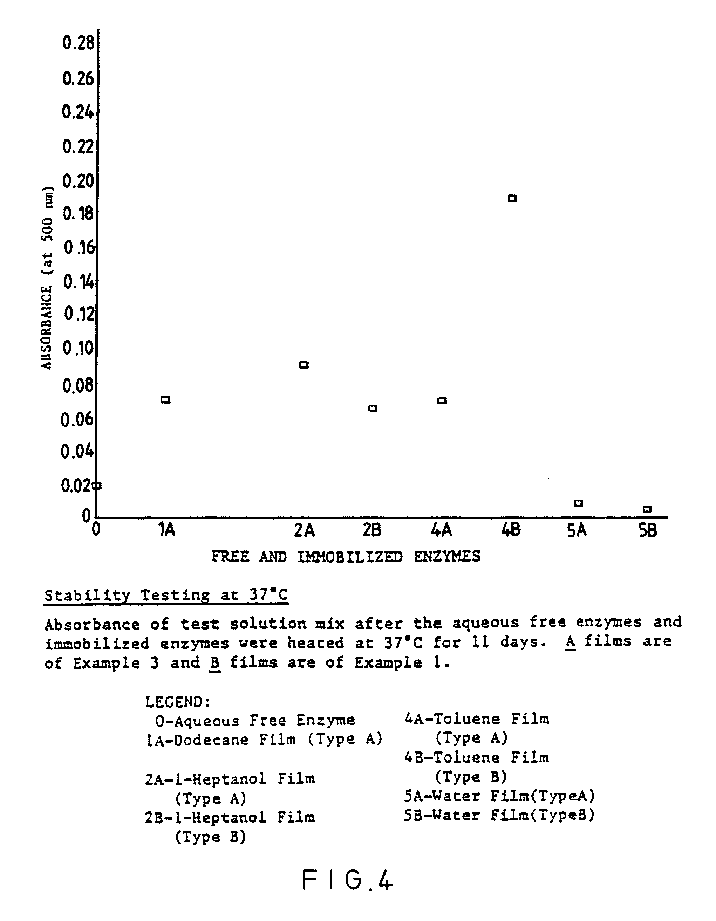

The solvent immobilized films along with aqueous buffered enzyme solution and aqueous enzyme immobilized films were incubated in an oven for 11 days at 37°C. The films and the control aqueous solution were analyzed as per example 3. The results are shown in Figure 4.

The trend is similar to that observed at 60°C. The aqueous enzyme solution in minimally active. Considering that the aqueous solution of enzymes (0.5 ml. volume) had approximately five times the enzymes as compared to immobilized enzyme discs (0.12 ml volume), the toluene film of Example 1 were at least 35 - 40 times as active as compared to free aqueous solution or aqueous immobilized films of Example 1. The toluene films of Example 3 were at least 15 times as active as compared to free native solutions. The other organic films with dodecane, and heptanol were also at least 15-20 times as active as the free enzyme solution.


Example 6:

An alternative approach of encapsulation of cholesterol oxidase and cholesterol esterase enzymes was attempted. Rather than pre-forming a membrane of polyacrylonitrile first and entrapping the enzymes afterwards as shown in examples 1 and 3, the enzymes can be in principle dissolved or dispersed directly in the polymer solution and the membrane formed thereafter would have enzymes coged in and encapsulated directly in the pore-walls, as for example taught by Stoy et al., U.S. Patent 4,110,529. However, in order to do this, the enzymes have to survive the rather hostile environment of polar organic solvent, at least for a short duration while the membrane is being cast and formed, and the solvent is being replaced by water.

To test the feasibility of this approach, the effect of 5 minutes of contact of cholesterol oxidase and cholesterol esterase with two different common solvents for polyacrylonitrile was investigated. The solvents were dimethylsulfoxide (DMSO), 55% aqueous solution of sodium thiocyanate, 2 mg. of cholesterol esterase and cholesterol oxidase powders were taken out in separate test tubes. Three sets of experiments were done: In one set, the powders were mixed in with 0.5 ml. DMSO solvent, in a second set, the powders were mixed in with 0.5 ml. sodium thiocyanate solvent and kept in contact with the same for 5 minutes. The last set served as a control.

After 5 minutes of contact time, the presence of activity of the enzymes was checked by the standard colorimetric method involving phenol-4- aminopyrine coupling reaction. For this, to each of test tube following reagents were added:

0.5 ml. of 3% sodium cholate in 0.1 M phosphate buffer at pH 7, 0.1 ml. of substrate solution (cholesterol oleate at 200 mg% in isopropyl alcohol to cholesterol esterase enzymes and cholesterol at 400 mg% in thesit-phosphate buffer to cholesterol exidase enzymes), 0.1 ml. of 3% Triton X-100 in D.I. water and 0.1 ml. of chromogen solution consisting of 4-amino antipyrine at 1.6 g%, phenol at 8 g% and sodium cholate at 12 g% in 0.1 M phosphate buffer. Additionally, to cholesterol esterase solution/suspension, about 10 mg. each of cholesterol oxidase and peroxidase enzyme powders were added whereas in cholesterol oxidase solution/suspension, 10 mg. of peroxidase were added. Immediately after these additions, the solutions/suspensions were mixed quickly and the resultant color was observed. Of the enzymes contacted with the solvents were active, they would produce a dark pink color very quickly. Such cola formation was observed only in the control buffer solution where the formation of color was instantaneous. Both cholesterol esterase and oxidase contacted either by DMSO or sodium thiocyanate solvents, failed to produce any color even after 10 minutes, indicating that the enzymes had lost all their activity rather quickly when contacted by these solvents.

This showed that such method of encapsulation is certainly too harsh for the enzymes tested and like many other fragile enzymes, they loose their activity quickly when subjected to such polar organic solvents.

TABLE 1:  **Effect of 60°C Heating on Enzyme Activity**
Type A films are those of example 3 and Type B films are those of example 1.  Increasing color intensity represents increasing activity.

| | |
|---|---|
| Aqueous enzyme solution and Type B aqueous film | No color |
| Type A aqueous film | Very faint pink |
| Type A 1-heptanol film and Type B 1-heptanol film | Light orange |
| Type B dodecane film | Light pink (turbid) |
| Type A dodecane film | Pink (clear) |
| Type A toluene film | Dark orangish pink |
| Type B toluene film | Very dark orangish pink |

**(Increasing color intensity)**

## Claims

1. An evenly dispersed, stabilized enzyme system comprising:
   (a) a solid carrier having a microporous structure;
   (b) at least one enzyme sequestered within the carrier pores at least at the carrier surface, the pores being filled with a water-immiscible organic liquid, the enzymes micro-environment comprising sufficient water to allow enzymatic activity in the organic liquid.

2. The system of claim 1 wherein pores of said microporous structure is in the range of 5 Angstoms to 10 microns in diameter.

3. The system of claim 1 or claim 2 wherein the solid carrier is a microporous membrane.

4. The system of claim 3 wherein the membrane is polyacrylonitrile.

5. The system of claim 1 wherein said enzyme is insoluble or sparingly soluble in water.

6. The system of claim 1 wherein the organic liquid has a dipole moment of less that 2.5 Debye units, and/or a boiling point of at least 40°C and/or a dielectric constant less than 20.

7. The system of any of claims 1 to 6 wherein at least 90% of the liquid content of the carrier is water immiscible organic liquid.

8. A method of preparing an evenly dispersed, stabilized enzyme system, comprising the steps of:
   (a) providing a solid carrier having a microporous structure, the pores being filled with an aqueous liquid;
   (b) contacting the carrier with an aqueous enzyme solution for a sufficient time to equilibrate with the aqueous liquid in the pores;

(c) causing a controlled, partial collapse of the pores to effectively entrap the enzyme within the pores; and,

(d) replacing the aqueous liquid in the pores with a non-polar organic liquid.

9. The method of claim 8 wherein pores of said microporous structure is in the range of 5 Angstoms to 10 microns in diameter.

10. The method of claim 8 or claim 9 wherein the solid carrier is a microporous membrane.

11. The method of claim 10 wherein the membrane is polyacrylonitrile.

12. The method of any of claims 8 to 11 wherein the organic liquid has a dielectric constant less than 20.

13. The method of any of claims 8 to 12 wherein at least 90% of the liquid content of the carrier is water immiscible organic liquid.

14. The method of any of claims 8 to 13, wherein the carrier is hydrophilic.

15. A method of determining the presence of a substance comprising:

(a) providing an evenly dispersed, stabilized enzyme system, having: (i) a solid carrier having a microporous structure;

(ii) at least one enzyme sequestered within the carrier pores at least at the carrier surface, the pores being filled with a water-immiscible organic liquid, the enzyme's micro-environment comprising sufficient water to allow enzymatic activity in the organic liquid;

wherein the enzyme catalyzes a reaction of the substance being assayed to produce a product;

(b) contacting the system with a solution containing the substance being assayed; and,

(c) determining the presence of the reaction product.

16. The method of claim 15 wherein the substance is cholesterol or cholesterol esters and the enzymes are cholesterol oxidase, cholesterol esterase and peroxidase.

17. A method of enzymatic production of a product from a substrate compound, comprising:

(a) providing an evenly dispersed, stabilized enzyme system, having:

(i) a solid carrier having a microporous structure;

(ii) at least one enzyme sequestered within the carrier pores at least at the carrier surface, the pores being filled with a water immiscible organic liquid, the enzyme's micro-environment comprising sufficient water to allow enzymatic activity in the organic liquid;

wherein the enzyme catalyzes conversion of the substrate compound into the product;

(b) contacting a solution containing the substrate compound with the system; and,

(c) recovering the product.

18. A method of sustained controlled release of an enzyme to an aqueous medium comprising:

(a) providing an evenly dispersed, stabilized enzyme system, having:

(i) a solid carrier having a microporous structure;

(ii) at least one enzyme sequestered within the carrier pores at least at the carrier surface, the pores being filled with a water immiscible organic liquid, the enzyme's micro-environment comprising sufficient water to allow enzymatic activity in the organic liquid;

wherein the enzyme is larger than the carrier pores, and the carrier is an aqueous fluid swell able polymer,

(b) exposing the system to the aqueous medium, wherein, the aqueous medium enters the pores, causing them to swell to release the enzyme.

```
┌─────────────────────────────────────┐
│   Membrane (containg water)          │
└─────────────────────────────────────┘
                │
                │         equilibrate with aqueous
                │         enzyme
                ▼
┌─────────────────────────────────────┐
│ Membrane (containing aqueous enzyme) │
└─────────────────────────────────────┘
                │
                │         partially dry to effect
                │         controlled collapse of pores
                ▼
┌─────────────────────────────────────┐
│     Reduced aqueous content of       │
│   Membrane and entrapping enzymes    │
└─────────────────────────────────────┘
                │
                │         contact with water miscible
                │         solvent
                ▼
┌─────────────────────────────────────┐
│   Membrane (containing enzyme) in    │
│     water-miscible organic liquid    │
└─────────────────────────────────────┘
                │
                │         contact with
                │         water-immiscible organic
                │         liquid
                ▼
┌─────────────────────────────────────┐
│ Stabilized, dispersed enzyme in membrane │
└─────────────────────────────────────┘
```

# F I G.1

Absorbance versus cholesterol concentration
for immobilized enzyme membrane.

# F I G.2

Absorbance versus Calf Serum as substrate for enzyme immobilized toluene film.

# FIG.3

Stability Testing at 37°C

Absorbance of test solution mix after the aqueous free enzymes and immobilized enzymes were heated at 37°C for 11 days. A films are of Example 3 and B films are of Example 1.

LEGEND:
 0-Aqueous Free Enzyme          4A-Toluene Film
1A-Dodecane Film (Type A)          (Type A)
                                4B-Toluene Film
2A-1-Heptanol Film                 (Type B)
    (Type A)                    5A-Water Film(TypeA)
2B-1-Heptanol Film              5B-Water Film(TypeB)
    (Type B)

F I G.4

# DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 265 253 (KINGSTON TECHNOLOGIES LTD PARTNERSHIP) <br> * Page 3, line 17 - page 4, line 38 * <br> — — — | 1-18 | C 12 N 11/08 <br> C 12 N 11/04 |
| X | PATENT ABSTRACTS OF JAPAN, vol. 11, no. 332 (C-455)[2779], 29th October 1987; <br> & JP-A-62 115 283 (SEITAI KINOU RIYOU KAGAKUHIN SHINSEIZOU GIJUTSU KENKYU KUMIAI) 26-05-1987 <br> * Abstract * <br> — — — | 1 | |
| A | US-A-4 705 753 (H.P. GREGOR et al.) <br> — — — — — | | |
| | | | **TECHNICAL FIELDS SEARCHED (Int. Cl.5)** |
| | | | C 12 N |

The present search report has been drawn up for all claims

| Place of search | Date of completion of search | Examiner |
|---|---|---|
| The Hague | 01 March 91 | REMPP G.L.E. |